## Europäisches Patentamt
## European Patent Office
## Office européen des brevets

(11) Veröffentlichungsnummer: **0 087 491**

**A1**

# EUROPÄISCHE PATENTANMELDUNG

(21) Anmeldenummer: **82101626.8**

(22) Anmeldetag: **03.03.82**

(51) Int. Cl.³: **A 61 M 5/00**
**A 61 M 5/31, G 04 B 47/00**
**A 61 J 1/00**

(43) Veröffentlichungstag der Anmeldung:
**07.09.83 Patentblatt 83/36**

(84) Benannte Vertragsstaaten:
**AT BE CH DE FR GB IT LI LU NL SE**

(71) Anmelder: **Petz Electro**
**Friesenstrasse 791**
**CH-3185 Schmitten(CH)**

(72) Erfinder: **Petz, Günter**
**Flachslander Strasse 8**
**D-8500 Nürnberg(DE)**

(74) Vertreter: **Göbel, Matthias, Dipl.-Ing.**
**Pruppacher Hauptstrasse 5-7**
**D-8501 Pyrbaum-Pruppach(DE)**

(54) **Diabetiker-Set.**

(57) Bei einem Diabetiker-Set mit einem durch einen kappenartigen Deckel verschließbaren Aufnahmeteil, der einen etuiförmigen Hohlkörper und einen Einsatz aufweist, bei dem der Einsatz Insulinflaschen fest und Injektionsspritzen abnehmbar haltert und im Aufnahmeteil auf der dem Deckel abgewandten Seite eine durch einen Deckel verschließbare Aufnahmekammer vorgesehen ist, ist zum Anzeigen der Zeiten für die Insulinspritzungen im Aufnahmeteil (24) und/oder einem der Deckel (28, 25) eine Zeitmeß- und/oder Zeitkontrolleinrichtung (39) angeordnet.

Fig. 1

EP 0 087 491 A1

DIPL.-ING. **M. GÖBEL**

PATENTANWALT

**0087491**

**8501 PYRBAUM-PRUPPACH**

PRUPPACHER HAUPTSTRASSE 5-7
TELEFON 09180/675
TELEGRAMM GOEPATENT PYRBAUM
TELEX 624407 GOEPA

BANKKONTEN:
VOLKSBANK NÜRNBERG 45233 BLZ 76090000
COMMERZBANK NÜRNBERG 8300907 BLZ 76040061

- 1 -

Petz Electro, CH 3185 Schmitten

Diabetiker-Set

Die Erfindung betrifft einen Diabetiker-Set mit einem durch einen kappenartigen Deckel verschließbaren Aufnahmeteil, der durch einen etuiförmigen Hohlkörper und einen Einsatz gebildet ist, bei dem der Einsatz Insulinflaschen fest und Injektionsspritzen abnehmbar haltert und im Aufnahmeteil auf der dem Deckel abgewandten Seite eine durch einen Deckel verschließbare Aufnahmekammer vorgesehen ist.

Bei einem etuiförmigen Diabetiker-Set sind nach dem Abziehen des Deckels die Insulinflaschen und die Injektionsspritzen im Aufnahmeteil zugänglich. Bei diesem Diabetiker-Set ist an dem der Entnahmeöffnung für Insulin abgewandten Ende eine Kammer vorgesehen, die der Aufnahme von Alkoholtüchern oder Zubehör dient und durch einen am Aufnahmeteil angeordneten Deckel verschließbar ist. Obwohl dieser Diabetiker-Set eine hygienische Halterung und Handhabung der Injektionsspritzen möglich macht, hat der Benutzer einen erheblichen Kontrollaufwand für die Spritzzeiten aufzubringen. Dies führt dazu, daß desöfteren Insulinspritzungen unbeabsichtigt verspätet erfolgen oder vergessen werden.

Es ist Aufgabe der Erfindung Maßnahmen zu schaffen,die dem Benutzer die Zeiten für die Insulinspritzungen anzeigen.

Erfindungsgemäß ist dies dadurch erreicht, daß im Aufnahmeteil und/oder einem der Deckel eine Zeitmeß- und/oder Zeitkontrolleinrichtung angeordnet ist. Die bevorzugt durch eine mechanische, elektrische oder elektronische Uhr gebildete Zeitmeß- und/oder Zeitkontrolleinrichtung, die zweckmäßig eine über eine Fensteröffnung einsehbare Anzeige aufweist, gibt dem Benutzer die Möglichkeit, die jeweilige Injektionszeit exakt abzulesen oder vorzuwählen. Hierdurch können die Insulininjektionen zu genau feststehenden Zeiten erfolgen. Es ist vorteilhaft, wenn die Zeitmeß- und Kontrolleinrichtung eine Weckeinrichtung aufweist, um so den Benutzer auf die Fälligkeit von Insulininjektionen aufmerksam zu machen. Auch ist eine rückzählbare Einrichtung (cont down) denkbar, die nach einem Spritzvorgang den folgenden Spritzvorgang, entsprechend einer wählbaren Zeit, anzählt.

In Ausgestaltung des Diabetiker-Sets ist vorgesehen, daß die Zeitmeß- und/oder Zeitkontrolleinrichtung zum einfachen und sicheren Erkennen eine durch Leuchtdioden (LED) oder durch Flüssigkristalle (LCD) gebildete Anzeige aufweist, während durch geeignete Betätigungsorgane die Zeitmeß- und/oder Zeitkontrolleinrichtung
verstell- und einstellbar ist.

Schließlich ist zum Schutze der Zeitmeß- und/oder Zeitkontrolleinrichtung, insbesondere der Anzeige noch vorgesehen, diese durch einen am Aufnahmeteil und/oder den
Deckeln geführten Schieber bzw. einer angelenkten Kappe
abzudecken.

Es entspricht dem Erfindungsgedanken, daß die Zeitmeß-
und/oder Zeitkontrolleinrichtung beliebig unmittelbar
am Aufnahmeteil oder dem betreffenden Deckel befestigt
ist oder in eine am Aufnahmeteil oder einem Deckel innen
ausgebildete und eine Fensteröffnung für die Anzeige
aufweisende Aufnahmekammer eingeschoben und festlegbar
ist.

Die Erfindung ist in der Zeichnung anhand von Ausführungsbeispielen erläutert. Es zeigen:

Figur 1    einen Diabetiker-Set  in Vorderansicht,

Figur 2    einen Diabetiker-Set  in Seitenansicht,
           teilweise im Schnitt,

Figur 3    ein Teilstück eines Diabetiker-Sets und

Figur 4    ein weiteres Teilstück eines Diabetiker-Sets.

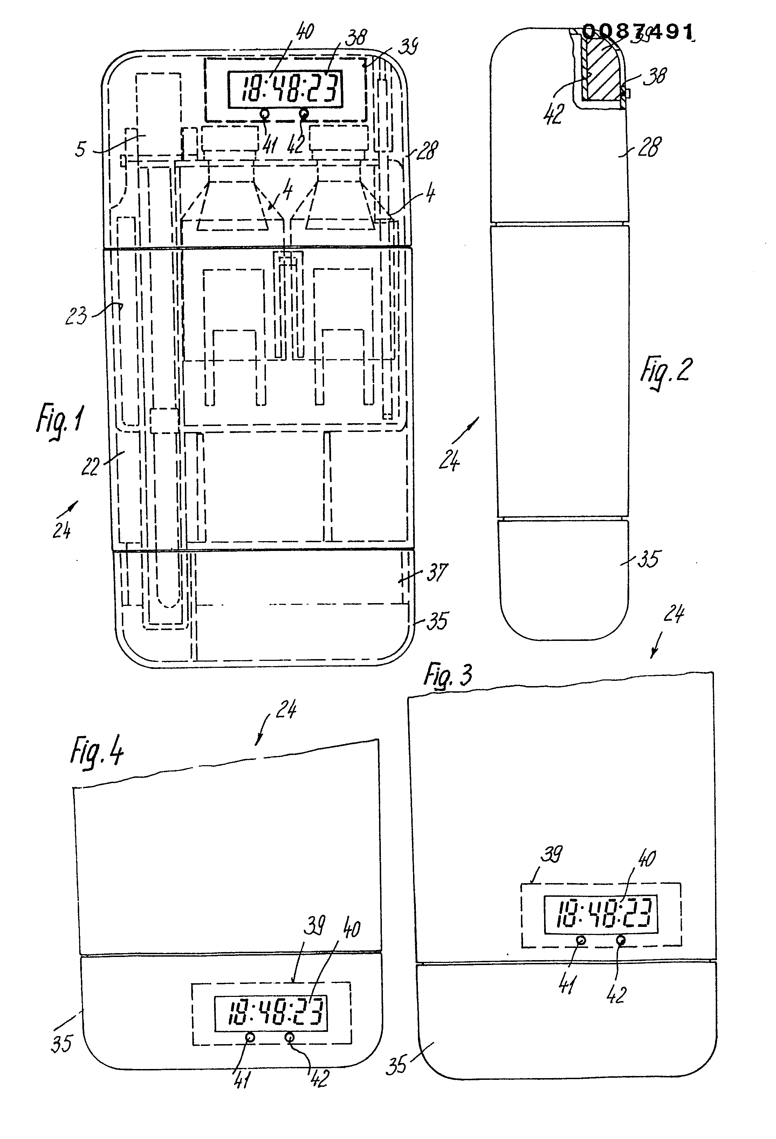

In den Figuren 1 bis 4 ist der etuiförmige Aufnahmeteil
für zwei Insulinflaschen 4 und zwei Injektionsspritzen
5 mit 24 bezeichnet. Der Aufnahmeteil 24 ist durch einen
Hohlkörper 22 und einem mit diesem verbundenen kastenförmigen Einsatz 23 gebildet und oben durch einen auf den Einsatz 23 aufschiebbaren Deckel 28 verschließbar. Das untere Ende des Aufnahmeteils 24 weist eine stirnseitig offene Kammer 37 für die Aufnahme von Alkoholtüchern bzw. Zubehör auf, die durch einen weiteren Deckel 35 verschließbar ist.

Beim Ausführungsbeispiel der Figuren 1 und 2 ist im Deckel 28 im Bereich hinter einer Fensteröffnung 38 eine Zeitmeß- und/oder Zeitkontrolleinrichtung 39 angeordnet, die zweckmäßig durch eine elektronische Uhr mit Digitalanzeige 40 und Betätigungsorganen 41, 42 für Verstell- und Einstellvorgänge gebildet ist. Die Zeitmeß- und/oder Zeitkontrolleinrichtung 39 kann mit oder ohne Weckeinrichtung (nicht gezeigt) ausgeführt sein. Die Zeitmeß- und/oder Zeitkontrolleinrichtung 39 erlaubt dieBenutzungszeiten des Diabetiker-Sets abzulesen und vermittels der Weckeinrichtung den Benutzer zu vorbestimmten, einstellbaren Zeiten an notwendige Insulinspritzvorgänge zu erinnern. Es entspricht der Erfindung, daß mittels der Zeitmeß- und/oder Zeitkontrolleinrichtung 39 auch eine selbsttätige Zeitrückzählung (cont down) über die Dauer von einstellbaren Zeitabschnitten mit anschließendem Wecsignal bewirkt werden kann. Zur beschädigungsfreien Unterbringung ist die Zeitmeß- und/oder Zeitkontrolleinrichtung 39 in eine Kammer 43 eingeschoben und in dieser, z.B. durch die Betätigungsorgane gehalten. Die Zeitmeß- und/oder Zeitkontrolleinrichtung 39 ist beim Ausführungsbeispiel der Figur 3 im Aufnahmeteil 24 und beim Ausführungsbeispiel der Figur 4 im Deckel 35 untergebracht. Es entspricht dem Erfindungsgedanken, daß als Zeitmeß- und/oder Zeitkontrolleinrichtung z.B. auch LED-Anzeigen-Bauelemente Anwendung finden können.

Patentansprüche

1. Diabetiker-Set mit einem durch einen kappenartigen Deckel verschließbaren Aufnahmeteil, der durch einen etuiförmigen Hohlkörper und einen Einsatz gebildet ist, bei dem der Einsatz Insulinflaschen fest und Injektionsspritzen abnehmbar haltert und im Aufnahmeteil auf der dem Deckel abgewandten Seite eine durch einen Deckel verschließbare Aufnahmekammer vorgesehen ist, dadurch gekennzeichnet, daß im Aufnahmeteil (24) und/oder einem der Deckel (28, 25) eine Zeitmeß- und/oder Zeitkontrolleinrichtung (39) angeordnet ist.

2. Diabetiker-Set nach Patentanspruch 1, dadurch gekennzeichnet, daß die Zeitmeß- und/oder Zeitkontrolleinrichtung (39) durch eine mechanische, elektrische oder elektronische Uhr gebildet ist.

3. Diabetiker-Set nach Patentanspruch 1 und 2, dadurch gekennzeichnet, daß die Zeitmeß- und/oder Zeitkontrolleinrichtung (39) eine über eine Fensteröffnung (38) einsehbare Anzeige (40) aufweist.

4. Diabetiker-Set nach Patentanspruch 1, 2 und 3, dadurch gekennzeichnet,daß die Zeitmeß- und/oder Zeitkontrolleinrichtung (39) eine durch Leuchtdioden gebildete Anzeige (40) aufweist.

5. Diabetiker-Set nach Patentanspruch 1, 2 und 3, dadurch gekennzeichnet, daß die Zeitmeß- und/oder Zeitkontrolleinrichtung (39) eine durch Flüssigkristalle gebildete Anzeige aufweist.

6. Diabetiker-Set nach Patentanspruch 1, dadurch gekennzeichnet, daß die Zeitmeß- und/oder Zeitkontrolleinrichtung (39) durch einen am Aufnahmeteil (24) und/oder den Eckeln (28, 35) geführten Schieber abdeckbar ist.

8. Diabetiker-Set nach Patentanspruch 3, dadurch gekennzeichnet, daß die Zeitmeß- und Zeitkontrolleinrichtung (39) durch eine am Aufnahmeteil (24) oder den Deckeln (28, 35) angelenkte Klappe verschließbar ist.

9. Diabetiker-Set nach Patentanspruch 1, dadurch gekennzeichnet, daß die Zeitmeß- und Zeitkontrolleinrichtung (39) als unabhängige Baueinheit ausgeführt und in eine am Aufnahmeteil (24) oder den Deckeln (28, 35) innen ausgebildete, eine Fensteröffnung aufweisende Aufnahmekammer (43) eingeschoben und lösbar festgelegt ist.

Fig. 1

Fig. 2

Fig. 3

Fig. 4

0087491

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| Y | US-A-2 740 516 (RENN)<br><br>* Spalte 1, Zeilen 16-22; Abbildung 1 *<br><br>--- | 1,2,4, 5,9 | A 61 M 5/00<br>A 61 M 5/31<br>G 04 B 47/00<br>A 61 J 1/00 |
| Y | EP-A-0 028 929 (STERWIN)<br><br>* Seite 1, Zeilen 1-9; Seite 3, Zeilen 12-14; Seite 7, Zeilen 3-6; Abbildung 8 *<br><br>--- | 1,2,4, 5 | |
| Y | FR-A-2 451 204 (SERVADIO)<br>* Seite 3, Zeilen 4-6; Seite 6, Zeilen 1-9; Anspruch 1, Abbildung 1 *<br><br>----- | 1,9 | |

| RECHERCHIERTE SACHGEBIETE (Int. Cl. ³) |
|---|
| A 61 J<br>A 61 M<br>G 04 B |

Der vorliegende Recherchenbericht wurde für alle Patentansprüche erstellt.

| Recherchenort | Abschlußdatum der Recherche | Prüfer |
|---|---|---|
| DEN HAAG | 06-10-1982 | DURAND-SMET J.E.J.S. |